# EUROPEAN PATENT SPECIFICATION

(11) **EP 1 052 985 B1**
(45) Date of publication and mention of the grant of the patent: **22.12.2004**
(21) Application number: 99905716.9
(22) Date of filing: 03.02.1999
(51) Int. Cl.: A61K 31/195, A61K 9/08, A61P 39/00

(54) **N,N'-BIS(2-HYDROXYBENZYL)ETHYLENEDIAMINE-N,N'-DIACETIC ACID AND SODIUM SALTS THEREOF FOR IRON CHELATING THERAPY**
N,N'-BIS(2-HYDROXYBENZYL)ETHYLENDIAMIN-N,N'-DIESSIGSÄURE UND NATRIUMSALZE DAVON ZUR EISENCHELATIERUNGSTHERAPIE
ACIDE N,N'-BIS(2- H YDROXYBENZYL)ETHYLENEDIAMINE-N, N'-DIACETIQUE ET SES SELS DE SODIUM DESTINES AU TRAITEMENT PAR CHELATION DU FER

(30) Priority: 04.02.1998 US 73603 P
(43) Date of publication of application: 22.11.2000
(73) Proprietor: University Of Florida Research Foundation, Inc., Gainesville, FL 32611-5500 (US)
(72) Inventor: BERGERON, Raymond, J., Jr., Gainesville, FL 32653 (US)
(74) Representative: Stuart, Ian Alexander
(86) International application number: PCT/US1999/002388
(87) International publication number: WO 1999/039706

(56) References cited:
- WO-A-95/16663
- WO-A-97/36885
- US-A- 5 057 302
- US-A- 5 227 474
- US-A- 5 534 241

## Description

### INTRODUCTION

### Technical Field

This invention relates to the use of N,N'-bis(2-hydroxybenzyl) ethylenediamine-N,N'-diacetic acid (HBED) in the manufacture of a composition for iron chelating therapy. In particular, the invention provides the subcutaneous use of HBED for treating a primate, in particular a human, that is afflicted with a disease state treatable with an iron chelating agent, particularly, iron overload, especially transfusional iron overload.

### Background

In humans and other primates iron is stored in the body in the form of ferritin and hemosiderin, which are protein complexes, and is transported in the plasma via another protein complex, namely transferrin. The manner in which iron is utilized is very efficient, but there is no specific mechanism that exists for the elimination of excess iron. Under conditions of iron overload the protein complexes become saturated with iron, resulting in excess iron being deposited in tissues which induces iron toxicity, and ultimately leads to peroxidative tissue damage. One common process by which iron overload occurs in humans is by repeated blood transfusions.

One way of treating iron overload is by administering an iron chelator. In principle, the chelator transforms the deposited iron back into a soluble form that is then capable of excretion. However, none of the available iron chelators employed for this purpose is ideal; the chelators have poor gastrointestinal absorption. or have low efficacy and/or undesirable side effects.

One iron chelator that overcomes some of these disadvantages, and is widely used in therapy, is deferoxamine B mesylate (DFO): In general DFO is capable of controlling excess body iron, and can prolong survival and prevent or ameliorate organ dysfunction in a person suffering from iron overload. However, DFO is far from an ideal treatment for iron overload. It is cumbersome. inefficient, expensive. and unpleasant to the patient. Because DFO is poorly absorbed from the gastrointestinal tract and rapidly eliminated from circulation, prolonged parenteral infusion is required for treatment; it is generally administered by a portable infusion pump for 9-12 hours daily. Not surprisingly, very few patients are capable of complying with such a demanding regimen. Additionally, DFO is very inefficient as an iron chelator, in that typically 5% or less of the compound administered binds iron.

Yet another disadvantage is the cost of DFO, which is a bacterial siderophore that is commercially produced by large scale fermentation of a strain of *Streptomyces pilosus,* a method of manufacture that is very expensive. In addition, almost all patients experience an allergic reaction to DFO, which is uncomfortable and sometimes very painful. The allergic reaction is thought to be caused by cytokines and/or other fermentation products formed during the fermentation process that are not completely removed during purification of the crude product mixture.

It would therefore be desirable to provide a method for treating iron overload that employs a safe and inexpensive alternative to DFO. Ideally, the iron chelator would not require prolonged parenteral infusion, would not be associated with an allergic reaction in patients, and would be a more efficient chelator of iron than DFO. It would also be an advantage if it were not required to be administered orally, because oral treatment requires the patient to ingest large amounts of chelator three or more times daily, and because there is concern that such ligands might exacerbate iron toxicity and themselves have undesirable side effects.

HBED is a compound known to be an iron chelator. U.S. Patent No. 3,758,540 discloses that iron chelates of HBED and other compounds are useful as a source of iron in plant nutrition. U.S. 4,528,196 discloses, on the basis of testing in rat and mouse screens, that HBED and its alkyl esters are orally active in the treatment of iron overload, and more active given orally than DFO given intraperitoneally. However, the rodent findings were not substantiated in higher animals. Testing in the *Cebus apella* monkey screen, which is known to be an excellent predictor of the behavior of chelators in humans, showed that DFO administered subcutaneously has significantly higher activity than HBED or its dimethyl ester given orally (Bergeron et al., Blood, 81:2166 (1993), Peter et al., "A Comparative Evaluation of Iron Chelators in a Primate Model", pp 373, Development of Iron Chelators for Clinical Use, Boca Raton, CRC Press (1994)). It has also been demonstrated that HBED, when given orally to human patients, provides iron excretion at a level that is ineffective for the treatment of iron overload (Grady et al., "Preliminary Results from a Phase I Clinical Trial of HBED", in the Development of Iron Chelators for Clinical Use, Boca Raton, CRC Press ( 1994), pp 395).

It was therefore totally unexpected, based upon the above findings, that HBED would prove to be a highly efficient compound for treating iron overload when administered subcutaneously. HBED upon subcutaneous administration does not require prolonged parenteral infusion for effective iron chelation and excretion, is not associated with an allergic reaction in patients, and is a more efficient chelator of iron than DFO.

### Objects of the Invention

An object of this invention is to provide a composition that may be used in an improved method for treating a disease state in primates, particularly humans, which disease state is treatable by an iron chelator.

It is a further object of this invention to provide a composition for the subcutaneous treatment of a disease state that is treatable by an iron chelator, particularly where the disease state is iron overload.

It is a further object of this invention to provide a process for making a subcutaneously administratable composition useful for treating a disease state treatable with an iron chelator, particularly where the disease state is iron overload.

It is a further object of this invention to provide a composition for treatment of a disease state treatable with an iron chelator, which does not require prolonged treatment of a patient (e.g., by parenteral infusion).

It is a further object of this invention to provide a treatment composition that minimizes allergic reactions to patients being treated for a disease state treatable by an iron chelator.

Other objects of the invention may be apparent to one of skill in the art upon reading the following specification and claims.

### Relevant Literature

U.S. Patents No. 3,758,540, 4,116,991, and 4,528,196 are of interest.

### SUMMARY OF THE INVENTION

The present invention relates to manufacture of a composition suitable for use in a method of treatment of a mammal having a disease state that is treatable by an iron chelator. The method comprises subcutaneous administration of a therapeutically effective amount of a compound of the formula (I) 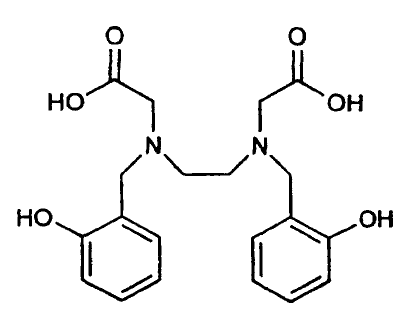 namely N,N'-bis(2-hydroxybenzyl)ethylenediamine-N,N'-diacetic acid (HBED) or a pharmaceutically acceptable salt thereof. The method is particularly suitable for treating iron overload in a primate.

Another aspect of this invention is a pharmaceutical composition comprising a pharmaceutically-acceptable excipient suitable for subcutaneous injection in combination with HBED as its monosodium or disodium salt.

Still another aspect of this invention is an article of manufacture that comprises a container containing a pharmaceutical composition comprising a pharmaceutically-acceptable excipient suitable for subcutaneous injection in combination with HBED as its monosodium or disodium salt, wherein the container holds a therapeutically effective amount of HBED and is associated with printed labelling instructions for subcutaneous administration of the composition to treat a disease state that is treatable by an iron chelator.

Still another aspect of this invention is a process for preparing a pharmaceutical composition, which process comprises combining a pharmaceutically-acceptable excipient suitable for subcutaneous injection with HBED as its monosodium or disodium.

Other aspects may become apparent upon further reading of this patent application.

### DESCRIPTION OF PRESENTLY PREFERRED EMBODIMENTS

### Method of Treatment

One aspect of this invention enables a method of treating mammals that have a disease state that is treatable by an iron chelator. The method comprises the subcutaneous administration of a therapeutically-effective amount of HBED or a pharmaceutically acceptable salt thereof. A disease state treatable by an iron chelator includes any disease of an individual mammal, particularly a primate such as a human, in which the individual shows improvement when an iron chelator is administered, selected from: malaria, cancer, HI-V infections, inflammatory bowel disease, host v. graft rejection, graft v. host rejection, reperfusion injury, neurological disorders, and iron overload. The disease state in which this method is most useful is "iron overload." It is characterized by greater than normal focal or generalized deposition of iron within body tissues (hemosiderosis). When such focal or generalized deposition is associated with tissue injury, with total body iron greater than about 15 grams, it is known as hemochromatosis. Generally, primary hemochromatosis results from a genetically determined error that results in increased absorption of iron from a normal diet, usually due to an autosomal recessive trait, but also includes atransferrinemia, thalassemia major, and y-linked hypochromic anemia. Secondary hemosiderosis or hemochromatosis shows increased parenteral iron intake such as through repeated transfusion (transfusional iron overload) or iron dextran taken intramuscularly. These may also be caused by increased iron absorption due to increased iron ingestion or from increased iron absorption from normal amount of dietary iron, but having anemia with erythroid hyperplasia and possibly through megadoses of Vitamin C. Focal hemosiderosis may be pulmonary, renal, or hepatic.

While the Merck Manual, 15th Edition, sets forth the classification of these conditions, differential diagnosis is difficult. Diagnosis will depend on the history of iron administration, the examination of relatives of the patient, the degree of iron overload, and the presence or absence of localizing signs. For primary hemochromatosis, which in its most common form affects three to eight people per thousand, the signs and symptoms are rare before middle age. Typical manifestations are cirrhosis of the liver, brown pigmentation of the skin, diabetes mellitus, and cardiomyopathy, which may be manifested by cardiomegaly, congestive failure, and arrhythmias or conduction disturbances. In the case of pituitary failure, testicle atrophy and loss of libido may be seen. Abdominal pain, arthritis and chondrocalcinosis occur less often. Focal hemosiderosis chiefly occurs in the lungs and kidneys. Pulmonary hemosiderosis may be due to recurrent pulmonary hemorrhage which occurs as an idiopathic entity, e.g., as part of Goodpasture's Syndrome. Diagnosis of these conditions may be found in Merck's Manual. Each of these conditions should be considered as a disease that is treatable by an iron chelator.

As mentioned herein before, the key to the treatment is the subcutaneous administration of the compound HBED as shown in Formula I hereinbefore, or a pharmaceutically acceptable salt thereof.

"Pharmaceutically-acceptable salt" means a salt that retains the biological effectiveness and properties of HBED, and that is not biologically or otherwise undesirable. HBED is capable of forming both acid and base salts by virtue of the presence of carboxylic acid and amino groups.
1. Pharmaceutically-acceptable base addition salts may be prepared from inorganic and organic bases. Salts derived from inorganic bases include, but are not limited to, the sodium, potassium, lithium, ammonium, calcium, and magnesium salts. Salts derived from organic bases include, but are not limited to, salts of primary, secondary and tertiary amines, substituted amines including naturally-occurring substituted amines, and cyclic amines, including isopropylamine, trimethylamine, diethylamine, triethylamine, tripropylamine, ethanolamine, 2-dimethylaminoethanol, tromethamine, lysine, arginine, histidine, caffeine, procaine, hydrabamine, choline, betaine, ethylenediamine, glycocyamine, N-alkylglucamines, theobromine, purines, piperazine, piperidine, and N-ethylpiperidine. It should also be understood that other carboxylic acid derivatives would be useful in the practice of this invention, for example carboxylic acid amides, including carboxamides, lower alkyl carboxamides, and di(lower alkyl) carboxamides.
2. Pharmaceutically-acceptable acid addition salts may be prepared from inorganic and organic acids. Suitable inorganic acids include hydrochloric acid, hydrobromic acid, sulfuric acid, nitric acid, and phosphoric acid. Suitable organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid and salicylic acid.

Preferred are the disodium salt, the monosodium salt, the dihydrochloride salt, and the monohydrochloride salt.

The term "treatment" as used herein covers any treatment of a disease treatable by an iron chelator in a mammal, particularly a human, and includes:
(i) preventing the disease from occurring in a subject which may be predisposed to the disease but has not yet been diagnosed as having it;
(ii) inhibiting the disease, i.e. arresting its development; or
(iii) relieving the disease, i.e. causing regression of the disease.

The method of administration of that has been found to be surprisingly effective is the subcutaneous administration of HBED or a pharmaceutically acceptable salt thereof. By subcutaneous administration it is meant that the drug in the form of an appropriate injectable composition is injected into the areola connective tissue just below the skin. The injection may be a solution, a suspension, or a formulation that provides a controlled release of the active entity. The subcutaneous administration will be done with excipients that are suitable for subcutaneous administration, which means that the excipients will have to meet USP considerations in being appropriate for injectable compositions. Thus, the composition will need to be sterile to avoid any complications due to insterility at the injection site.

The amount of the active ingredient that will be present in the composition to be injected and that will be injected is a therapeutically-effective amount, that is, an amount which is sufficient to result in successful treatment as defined above when administered to a mammal exhibiting a disease state treatable by an iron chelator, e.g., a disease state of iron overload. The therapeutically effective amount will vary depending on the subject and disease state being treated, the severity of the affliction and the manner of administration, and may be determined routinely by one of ordinary skill in the art in light of the disclosure of this specification. The typical daily dose of HBED varies according to individual needs, the severity of condition to be treated and the specific salt chosen. Generally, about 5 micromoles (µM) to about 500µM of the active moiety, i.e., HBED, will be administered to a patient on a per kilogram (Kg) basis. Suitable doses are in the general range from about 1 to about 200 mg/Kg body weight of the recipient per day, preferably in the range from about 5 to about 100 mg/Kg, most preferably in the range from about 10 to about 80 mg/Kg.

### Pharmaceutical Compositions of the Invention

Broadly, the compositions of the invention are the combination of HBED as its monosodium or disodium salt with a pharmaceutical excipient that is suitable for injection. Generally, the compositions of the invention or prepared by the use of the invention may fall into one of six (6) categories:
1. a solution that is ready for injection,
2. a dry soluble composition that is ready to be combined with a solvent just prior to use,
3. a suspension ready for injection,
4. a dry insoluble composition ready to be combined with the vehicle just prior to use,
5. an emulsion ready for injection, and
6. a liquid concentrate ready for dilution prior to administration.
In preparing a composition for subcutaneous administration strict attention must be paid to tonicity adjustment to avoid irritation of the plentiful nerve endings in this anatomical area.

The subcutaneous injections are generally quite dilute and the component present in the highest proportion is the vehicle. The vehicle normally has no therapeutic activity and is nontoxic, but presents the active constituent to the body tissues in a form appropriate for absorption. Absorption normally will occur most rapidly and completely when HBED or the appropriate salt or ester is presented as an aqueous solution. However, modification of the vehicle with water-miscible liquids or substitution with water-immiscible liquids can affect the rate of absorption. Absorption from a suspension may be affected by such factors as the viscosity of the vehicle, its capacity for wetting the solid particles, the solubility equilibrium produced by the vehicle, and the distribution coefficient between the vehicle and the aqueous body system. Preferably, the vehicle of greatest value for this subcutaneous composition is water that meets the USP specification for water for injection. Generally, water of suitable quality for compounding will either be prepared by distillation or reverse osmosis to meet these USP specifications. The appropriate specifications are spelled out in Remington: The Science and Practice of Pharmacy" 19th Ed. at pps. 1526-1528. In preparing the compositions which are suitable for subcutaneous injection, one can use aqueous vehicles, water-miscible vehicles, and nonaqueous vehicles. Certain aqueous vehicles are recognized officially because of their valid use in parenterals generally, and subcutaneous injectables specifically. These vehicles are used as isotonic vehicles to which the drug may be added at the time of administration. An isotonic solution is one that causes cells to neither shrink nor swell when it comes in contact with the cells. These are prepared so the additional osmotic effect of the drug will not be enough to produce any discomfort when subcutaneously administered. These aqueous vehicles useful in the invention include sodium chloride injection, Ringer's injection, dextro injection, dextro and sodium chloride injection, and lactated Ringer's injection. Preferably, a phosphate buffered aqueous solution of the monosodium salt of HBED is used.

Water-miscible vehicles are also useful in the formulation of the subcutaneous composition of this invention. These solvents are used primarily to affect the solubility of the various forms of the HBED and to reduce hydrolysis of the compound. The most important solvents in this group are ethyl alcohol, polyethylene glycol and propylene glycol.

Nonaqueous vehicles may also be used for a HBED salt that is relatively water-insoluble, e.g., the dihydrochloride salt of HBED. These vehicles include fixed oils, for example, those of a vegetable origin to allow for proper metabolism. For a USP subcutaneous injection injectable composition, the USP specifies limits for the degree of unsaturation and free fatty acid content. The oils most commonly used are corn oil, cottonseed oil, peanut oil, and sesame oil. Also useful are certain, more recently developed neutral oils that are esters of medium-chain fatty acids, and are also called fractionated coconut oil. Medium chain fatty acids, e.g. those of 8 to 10 carbon atoms, include compounds referred to as MIGLYOL®, which are sold by Dynamit Nobel. Five types of MIGLYOL® identified as MIGLYOL® 810, 812, 818, 829, and 840 are useful. These are more fully described in trade literature from Dynamit Nobel. A useful formulation is one containing about 33% wt./vol. of the dihydrochloride salt of HBED in a MIGLYOL vehicle. Certain other esters are also useful as nonaqueous vehicles, for example, triglycerides and propylene glycol diesters.

Additional substances may be included in the subcutaneous injectable compositions of this invention to improve or safeguard the quality of the composition. Thus, an added substance may affect solubility, provide for patient comfort, enhance the chemical stability, or protect preparation against the growth of microorganisms. Thus, the composition may include an appropriate solublizer, substances to make a solution isotonic, substances to act as antioxidants or buffers, and substances that act as a preservative to prevent the growth of microorganisms. These substances will be present in an amount that is appropriate for their function, but will not adversely affect the action of the composition as a treatment for iron overload. Examples of appropriate antimicrobial agents include thimerasol, benzethonium chloride, benzalkoniumchloride, phenol, methyl *p*-hydroxybenzoate and propyl *p*-hyrodxybenzoate. Appropriate buffers and antioxidants may be found in Remington at p. 1529.

Generally the sterile, subcutaneously injectable composition of this invention will comprise about .01% by wt. to about 50% by wt. of HBED or a corresponding salt, with the remainder being the appropriate excipient or excipients.

### Method of Preparation

Another aspect of this invention flows from the discovery of the unusual results obtained by subcutaneous administration of the drug. This aspect is a method of preparing a pharmaceutical composition suitable for subcutaneous administration which process comprises combining HBED as its monosodium or disodium salt with a pharmaceutical excipient suitable for subcutaneous administration under conditions that are effective to provide a sterile composition suitable for subcutaneous administration. Thus, in preparing the compositions of this invention, care must be taken to ensure the final composition is sterile and suitable for subcutaneous injection. The process will generally follow currently approved good manufacturing procedures (GMP) in order to result in the desired subcutaneous product. When employing aqueous vehicles the USP water for injection must be used for preparing the composition itself and preferably is used in preparing the equipment for preparing the composition. The general considerations for preparing subcutaneous preparations may be found in Remington: The Science and Practice of Pharmacy, 19th Ed. Chap. 87.

### Article of Manufacture

Another aspect of this invention is an article of manufacture that comprises a container holding a composition which is suitable for injection in combination with printed labelling instructions providing a discussion of how to administer the composition subcutaneously. A therapeutically-effective amount of the composition will be contained in any suitable container that will not significantly interact with the composition and will be labelled with the appropriate labelling that indicates it will be for subcutaneous use only. Associated with the container will be the labelling instructions as approved by the FDA in the process of gaining approval for the use of HBED as its monosodium or disodium salt for use in treating the disease state. The labelling will be consistent with the method of treatment as described hereinbefore. The container which holds the composition of this invention may be a container having a liquid composition suitable for injection which has an appropriate needle for injection and a syringe so that the patient, doctor, nurse, or other practitioner, can administer the drug. Representative examples of a suitable container include those used with the Wyeth-Ayerst TUBEX® closed injection system, with the SmithKline Beecham injectable product COMPRAZINE®, and with the Hoechst-Rousell injectable product LASIX® prefilled syringe. Alternatively, the composition may be a dry composition containing a soluble or suspendable version of the salt of HBED, to be combined with an aqueous or nonaqueous vehicle to dissolve or suspend the composition. Alternatively, the container may have a suspension in a liquid or may be an insoluble version of the HBED salt for combination with a vehicle in which the insoluble version will be suspended. Appropriate containers are discussed in Remington: Chapter 37. Whatever the particular container is, the labelling is a key aspect of the invention in that ultimately the product cannot be marketed unless it is approved by the Food and Drug Administration in the United States.

### EXAMPLES

The following examples are given as nonlimiting representations of various aspects of the invention to enable one of ordinary skill in the art to make and use the invention. In the examples, deferoxamine B (DFO) in the form of the methanesulfonate salt (trademark: DESFERAL) was manufactured by Ciba-Geigy Ltd. (Basel, Switzerland). HBED dihydrochloride dihydrate was obtained from Strem Chemical Co. (Newburyport, MA). Cremophor RH-40 was obtained from BASF (Parsippany, NJ).(CREMOPHOR is a trademark.) Sprague-Dawley rats were purchased from Charles River (Wilmington, MA). *Cebus apella* monkeys were obtained from World Wide Primates (Miami, FL). All reagents and standard iron solutions were obtained from Aldrich Chemical Co. (Milwaukee, WI).

### EXAMPLE 1

This example explains how to handle rats and *Cebus apella* monkeys for testing to determine the effectiveness of the process and composition of this invention.

### A. Cannulation of bile duct in rats

Male Sprague-Dawley rats averaging 400 g were housed in Nalgene plastic metabolic cages during the experimental period and given free access to water. The animals were anesthetized using sodium pentobarbital (55 mg/Kg) given intraperitoneally. The bile duct was cannulated, using 22-gauge polyethylene tubing, about 1 cm from the duodenum. The cannula was inserted about 2 cm into the duct, and once bile flow was established, the cannula was tied snugly in place. A skin tunneling needle was inserted from the shoulder area around to the abdominal incision. The cannula was threaded through the needle until it emerged from the shoulder opening. The cannula was then passed from the rat to the swivel inside a metal torque-transmitting tether, which was attached to a rodent jacket around the animal's chest. The cannula was directed from the rat to a Gilson micro fraction collector by a fluid swivel mounted above the metabolic cage. This system allowed the animal to move freely in the cage while continuous bile samples were being collected. Bile samples were collected at 3 hour intervals for 24 hours. Urine samples were taken every 24 hours. Sample collection and handling were as previously described. (Bergeron et al., Journal of Medicinal Chemistry 34:2072 (1991); Bergeron et al., Blood 79:1882 (1992)).

### B. Iron loading of Cebus apella monkeys

After intramuscular anesthesia with ketamine, an intravenous infusion was started in a leg vein. Iron dextran was added to approximately 90 mL of sterile normal saline and administered to the animals by slow infusion at a dose of 200 to 300 mg iron per Kg body weight over 45 to 60 minutes. Two to three infusions, separated by between 10 and 14 days, were necessary to provide about 500 mg iron per Kg body weight. After administration of iron dextran, the serum transferrin iron saturation rose to 70-80%. The serum half-life of iron dextran in humans is 2.5 to 3.0 days. At least twenty half-lives, sixty days, passed before using any of the animals in experiments evaluating iron-chelating agents.

### C. Iron-balance studies in Cebus apella monkeys

Seven days before the administration of the drug, the animals were placed in metabolic cages and started on a low-iron liquid diet. The monkeys were maintained on the low-iron liquid diet for the duration of the experiment. They were given food according to their body weight and intake was very carefully monitored.

Three days prior to drug administration, day -2 to day 0, baseline iron intake and output values were measured. This same measurement was made for day +1 to day +3. The total amount of iron intake was compared with the total amount of iron excreted.

### D. Primate fecal and urine samples

Fecal and urine samples were collected at 24 hour intervals. The collections began four days prior to the administration of the test drug and continued for an additional five days after the drug was given. Fecal samples were assayed for the presence of occult blood, weighed, and mixed with distilled deionized water prior to autoclaving for 30 minutes. The mixture was then freeze-dried, and a known portion of the powder was mixed with low-iron nitric acid and refluxed for 24 hours. Once any particulate matter in the digested samples was removed by centrifugation, iron concentrations were determined by flame atomic absorption. Monkey urine samples were acidified and reconstituted to initial volume after sterilization, if necessary.

### EXAMPLE 2

This example explains how formulations are prepared and administered for the process (s.c. administration) and composition of this invention as compared to the known process (oral administration) of the art.

### A. Drug preparation and administration

DFO was administered to the rats at a dose of 150 moles/Kg in 40%Cremophor RH-40, HBED was administered subcutaneously (s.c.) and orally by gavage (p.o.) to the rats at a dose of 150 moles/Kg in a phosphate buffer.

In the primates, DFO was administered s.c. in sterile water for injection at a dose of 150 moles/Kg, while HBED was first dissolved in a phosphate buffer and given s.c. in 40% wt./vol. Cremophor RH-40 at a dose of 75 or 150 moles/Kg. In addition, to more closely mimic clinical applications in patients, HBED was also given to the monkeys s.c. at a dose of 150 moles/Kg in a phosphate buffer, no Cremophor vehicle was used.

### B. Calculation of iron chelator efficiency

The efficiency of each chelator was calculated on the basis of a 1:1 ligand-iron complex. In the monkeys, the numbers were generated by averaging the iron output for four days prior to the administration of the drug, subtracting these numbers from the two-day iron clearance after the administration of the drug, and then dividing by the theoretical output; the result is expressed as a percent. The efficiencies in the rodent model were calculated by subtracting the iron excretion of control animals from the iron excretion of treated animals. This number was then divided by the theoretical output; the result is expressed as a percent.

### C. Statistical analysis

Data are presented as the mean ± standard error of the mean. For comparisons of the means of two groups, the two-sample t-test (without the assumption of equality of variances) was used for analyzing the rodent data, while the primate data were analyzed using a paired t-test. All tests were two-tailed, and a significance level of P <0.05 was used.

### EXAMPLE 3

This example compares the effectiveness in rats of subcutaneously administered HBED in accordance with the invention vs. orally administered HBED and subcutaneously administered DFO. The comparators include the timeline of iron excretion induced by the iron chelators and the peak amounts of iron excreted.

These studies were conducted in non-iron overloaded, bile-duct cannulated rats. The chelators were administered at a dose of 150 moles/Kg body weight. Groups of rats were given DFO by s.c. injection (n = 6) or HBED orally by gavage (n = 4) or s.c. injection (n = 3). The time course of the mean biliary iron excretion induced by the chelators in each group of rats, is expressed as g Fe/Kg body weight. The peak amounts of iron excreted withs.c. HBED were more than two-fold greater than the peak iron excretion after either s.c. DFO or HBED given by gavage (P < 0.05 at t = 3 h and P < 0.01 at t = 6 h). The iron excretion induced by the chelators in each group of rats is expressed as the net mean amount of iron excreted in the urine and in the bile (g Fe/Kg body weight) and as the efficiency of iron chelation (net iron excretion/total iron-binding capacity of chelator administered, expressed as a percent).

Subcutaneous DFO induced the excretion of 209 ± 59 g Fe/Kg body weight and was found to have an efficiency of 2.5 ± 0.7% (range, 1.7 to 3.7%), with about 74% of the chelator-induced iron excreted through the bile and about 26% through the urine. Compared to s.c. DFO, HBED given orally by garage resulted in a two-fold greater iron excretion of 436 ± 176 g Fe/Kg body weight (P < 0.02); the efficiency was 5.2±2.1% (range, 3.5 to 8.3%), with about 97% of the chelator-induced iron excretion through the bile and about 3% through the urine. HBED given to the rodents by s.c. injection was more than three times as effective in inducing iron excretion as DFO administered s.c. (P < 0.001), inducing the excretion of 679 ± 8 g Fe/Kg body weight with an overall efficiency of 8.1±0.1% (range, 7.9 to 8.2%). About 83% of the iron was excreted in the bile and 17% in the urine. No untoward side effects were noted in the rodents.

### EXAMPLE 4

This example compares the effectiveness in *Cebus apella* monkeys of subcutaneously administered HBED in accordance with the invention vs. orally administered HBED and subcutaneously administered DFO. The comparators include the timeline of iron excretion induced by the iron chelators and the peak amounts of iron excreted.

### Chelator-induced iron excretion in Cebus apella monkeys

The studies were conducted with *Cebus apella* monkeys to which had been previously administered intravenous iron dextran to provide about 500 mg iron per Kg body weight, as described above. Groups of monkeys (n = 6 in each group) were given s.c. injections of either DFO or HBED. DFO in aqueous solution was given s.c. at a dose of 150 moles/Kg and induced the excretion of 435 ± 115 g Fe/Kg body weight and was found to have an efficiency of 5.1 ± 1.3% (range, 3.3 to 6.6%), with about 65% of the chelator-induced iron excretion in the stool and about 35% in the urine. In our initial experiments, the HBED was dissolved in a phosphate buffer and administered in 40% wt./vol. Cremophor RH-40, a polyethoxylated castor oil used as a vehicle for compounds with poor aqueous solubility, because of the low water solubility of the HBED dihydrochloride dihydrate. HBED-Cremophor was administered by s.c. injection at doses of 75 or 150 moles/Kg. At the dose of 75 moles/Kg, HBED-Cremophor induced the clearance of 793 ± 410 g/Kg of iron and had an efficiency of 18.4 ± 9.1% (range, 7.4 to 27.8%). Most of the iron (92%) was excreted in the feces, while 8% was excreted in the urine. At a dose of 150 moles/Kg, HBED-Cremophor induced the clearance of 1349 ± 475 g/Kg, an efficiency of 16.1 ± 5.6% (range, 9.3 to 23.0%). Once again, the majority of the iron, 90%, was excreted in the feces; 10% was found in the urine.

Finally, in order to more closely mimic potential clinical applications, HBED (as the monosodium salt) was also given s.c. to the same group of monkeys used in the preceding experiments at a dose of 150 moles/Kg in a phosphate buffer; noCremophor vehicle was used. Once again, subcutaneous HBED induced the excretion of about twice as much iron as DFO, 899 ± 193 g Fe/Kg body weight (P < 0.001), and was found to have an efficiency of 10.7 ± 2.3% (range, 8.3 to 13.8%), with about 92% of the chelator-induced iron excretion in the stool and about 8% in the urine. At the dose of 150 moles/Kg no significant difference (P > 0.2) was found between the mean net iron excretion induced by HBED prepared in phosphate buffer or in Cremophor. The mean iron excretion induced by DFO given s.c. in an aqueous solution and HBED administered s.c. in a buffer at a dose of 150 moles/Kg body weight are expressed as the mean net amount of iron excreted in the urine and in the feces ( g Fe/Kg body weight) and as the efficiency of ironchelation. The results of this study can be compared to the results of a previously published study of the oral administration of HBED in a phosphate buffer to a group of *Cebus apella* monkeys with a similar magnitude of iron overload. Oral HBED induced the excretion of only 50 ± 44 g Fe/Kg body weight and was found to have an efficiency of 0.5 ± 0.5% (range, 0.1 to 1.1%), with about 56% of the chelator-induced iron excretion in the stool and about 44% in the urine. No adverse effects of chelator administration were noted in the monkeys; all hematological and biochemical tests remained within normal ranges.

### EXAMPLE 5

To prepare an aqueous, buffered, subcutaneously-injectable composition of this invention 60 mg of the disodium salt of HBED are dissolved in 1 ml sterile aqueous phosphate buffer suitable for injection to give a solution of a composition of this invention.

### EXAMPLE 6

To prepare a non-aqueous subcutaneously injectable composition of this invention, 60 mg of the dihydrochloride dihydrate salt are dissolved in 1 ml sterile CREMOPHOR RH-40, a polyethyoxylated castor oil, to give composition of this invention.

## Claims

1. A pharmaceutical composition comprising a pharmaceutically-acceptable excipient suitable for subcutaneous injection in combination with a therapeutically effective amount of the monosodium or disodium salt of N,N'-bis(2-hydroxybenzyl)ethylenediamine-N,N'-diacetic acid.

2. The composition of claim 1, wherein the compound is combined with a pharmaceutically-acceptable excipient as an injectable solution.

3. The composition of claim 1, wherein the compound is combined with a pharmaceutically-acceptable excipient as an injectable suspension.

4. The composition of claim 1, wherein the compound is combined with a pharmaceutically-acceptable excipient as an aqueous composition.

5. The composition of claim 1, wherein the compound is combined with a pharmaceutically-acceptable excipient as a non-aqueous composition.

6. Use of a pharmaceutically-acceptable excipient suitable for subcutaneous injection and N,N'-bis(2-hydroxybenzyl)ethylenediamine-N,N'-diacetic acid or a pharmaceutically-acceptable salt thereof in the manufacture of a composition for treating a mammal having a disease state that is treatable by an iron chelator and is selected from malaria, cancer, HIV infections, inflammatory bowel disease, host v. graft rejection, graft v. host rejection, reperfusion injury, neurological disorders, and iron overload, wherein a therapeutically effective amount of the composition is to be administered by subcutaneous injection.

7. The use of claim 6, wherein the disease state is iron overload.

8. The use of claim 7, wherein the disease state is primary hemochromatosis.

9. The use of claim 7, wherein the disease state is secondary hemochromatosis.

10. The use of claim 7, wherein the disease state is focal hemosiderosis.

11. An article of manufacture that comprises a container containing a pharmaceutical composition comprising a pharmaceutically-acceptable excipient suitable for subcutaneous injection in combination with the monosodium or disodium salt of N,N'-bis(2-hydroxybenzyl)ethylenediamine-N,N'-diacetic acid, wherein the container holds a therapeutically-effective amount of the salt and is associated with printed labelling instructions for administration of the composition to treat a disease state that is treatable by an iron chelator, said disease being selected from malaria, cancer, HIV infections, inflammatory bowel disease, host v. graft rejection, graft v. host rejection, reperfusion injury, neurological disorders, and iron overload.

12. The article of manufacture of claim 11, wherein the disease state is an iron overload state that is primary hemochromatosis, secondary hemochromatosis, or focal hemosiderosis.

13. The article of manufacture of claim 11, wherein the compound is combined with a pharmaceutically-acceptable excipient as an injectable solution, an injectable suspension, an aqueous composition, or a non-aqueous composition.

14. A process for preparing a pharmaceutical composition, which process comprises combining a pharmaceutically-acceptable excipient suitable for subcutaneous injection with the monosodium or disodium salt of N,N'-bis(2-hydroxybenzyl)ethylenediamine-N,N'-diacetic acid.

15. The process of claim 14, wherein the salt is the disodium salt.

16. The process of claim 14, wherein the salt is the monosodium salt.

17. The process of claim 14, 15 or 16, wherein the salt is combined with a pharmaceutically-acceptable excipient as an injectable solution, an injectable suspension, an aqueous composition, or a non-aqueous composition.

18. The monosodium salt of N,N'-bis(2-hydroxybenzyl)ethylenediamine-N,N'-diacetic acid.

19. The disodium salt of N,N'-bis(2-hydroxybenzyl)ethylenediamine-N,N'-diacetic acid.

20. A method of preparing a composition useful for treating a mammal having a disease state that is treatable by an iron chelator, said disease being selected from malaria, cancer, HIV infections, inflammatory bowel disease, host v. graft rejection, graft v. host rejection, reperfusion injury, neurological disorders, and iron overload which method comprises combining a pharmaceutically-acceptable excipient suitable for subcutaneous injection with the monosodium or disodium salt of N,N'-bis(2-hydroxybenzyl)ethylenediamine-N,N'-diacetic acid.

## Patentansprüche

1. Pharmazeutische Zusammensetzung, umfassend einen pharmazeutisch annehmbaren Exzipienten, der zur subkutanen Injektion in Kombination mit einer therapeutisch wirksamen Menge des Mononatrium- oder Dinatriumsalzes von N,N'-Bis(2-hydroxybenzyl)ethylendiamin-N,N'-diessigsäure geeignet ist.

2. Zusammensetzung nach Anspruch 1, worin die Verbindung mit einem pharmazeutisch annehmbaren Exzipienten als injizierbare Lösung kombiniert ist.

3. Zusammensetzung nach Anspruch 1, worin die Verbindung mit einem pharmazeutisch annehmbaren Exzipienten als injizierbare Suspension kombiniert ist.

4. Zusammensetzung nach Anspruch 1, worin die Verbindung mit einem pharmazeutisch annehmbaren Exzipienten als wässrige Zusammensetzung kombiniert ist.

5. Zusammensetzung nach Anspruch 1, worin die Verbindung mit einem pharmazeutisch annehmbaren Exzipienten als nichtwässrige Zusammensetzung kombiniert ist.

6. Verwendung eines pharmazeutisch annehmbaren Exzipienten, der zur subkutanen Injektion geeignet ist, und von N,N'-Bis(2-hydroxybenzyl)ethylendiamin-N,N'-diessigsäure oder eines pharmazeutisch annehmbaren Salzes davon bei der Herstellung einer Zusammensetzung zur Behandlung eines Säugetiers mit einer Krankheit, die durch einen Eisenchelatbildner behandelt werden kann, ausgewählt aus Malaria, Krebs, HIV-Infektionen, Darminfektionskrankheiten, Transplantatabstoßung, Transplantat-Wirt-Reaktionen, Reperfusionsschäden, neurologischen Störungen und Eisenüberladung, worin eine therapeutisch wirksame Menge der Zusammensetzung durch subkutane Injektion verabreicht werden soll.

7. Verwendung nach Anspruch 6, worin die Krankheit Eisenüberladung ist.

8. Verwendung nach Anspruch 7, worin die Krankheit primäre Hämochromatose ist.

9. Verwendung nach Anspruch 7, worin die Krankheit sekundäre Hämochromatose ist.

10. Verwendung nach Anspruch 7, worin die Krankheit fokale Hämosiderose ist.

11. Fabrikat, umfassend einen Behälter, der eine pharmazeutische Zusammensetzung enthält, die einen pharmazeutisch annehmbaren Exzipienten, der zur subkutanen Injektion geeignet ist, in Kombination mit dem Mononatrium- oder Dinatriumsalz von N,N'-Bis(2-hydroxybenzyl)ethylendiamin-N,N'-diessigsäure umfasst, worin der Behälter eine therapeutisch wirksame Menge des Salzes enthält und mit einem Beipackzettel mit Anleitungen für die Verabreichung der Zusammensetzung zur Behandlung einer Krankheit geliefert wird, die durch einen Eisenchelatbildner behandelt werden kann, ausgewählt aus Malaria, Krebs, HIV-Infektionen, Darminfektionskrankheiten, Transplantatabstoßung, Transplantat-Wirt-Reaktionen, Reperfusionsschäden, neurologischen Störungen und Eisenüberladung.

12. Fabrikat nach Anspruch 11, worin die Krankheit eine Eisenüberladung ist, nämlich primäre Hämochromatose, sekundäre Hämochromatose oder fokale Hämosiderose.

13. Fabrikat nach Anspruch 11, worin die Verbindung als injizierbare Lösung, injizierbare Suspension, wässrige Zusammensetzung oder nichtwässrige Zusammensetzung mit einem pharmazeutisch annehmbaren Exzipienten kombiniert ist.

14. Verfahren zur Herstellung einer pharmazeutischen Zusammensetzung, wobei das Verfahren das Kombinieren eines pharmazeutisch annehmbaren Exzipienten, der zur subkutanen Injektion geeignet ist, mit dem Mononatrium- oder Dinatriumsalz von N,N'-Bis(2-hydroxybenzyl)ethylendiamin-N,N'-diessigsäure umfasst.

15. Verfahren nach Anspruch 14, worin das Salz das Dinatriumsalz ist.

16. Verfahren nach Anspruch 14, worin das Salz das Mononatriumsalz ist.

17. Verfahren nach Anspruch 14, 15 oder 16, worin das Salz als injizierbare Lösung, injizierbare Suspension, wässrige Zusammensetzung oder nichtwässrige Zusammensetzung mit einem pharmazeutisch annehmbaren Exzipienten kombiniert wird.

18. Mononatriumsalz von N,N'-Bis(2-hydroxybenzyl)ethylendiamin-N,N'-diessigsäure.

19. Dinatriumsalz von N,N'-Bis(2-hydroxybenzyl)ethylendiamin-N,N'-diessigsäure.

20. Verfahren zur Herstellung einer Zusammensetzung, die zur Behandlung eines Säugetiers mit einer Krankheit nützlich ist, die durch einen Eisenchelatbildner behandelt werden kann, wobei die Krankheit aus Malaria, Krebs, HIV-Infektionen, Darminfektionskrankheiten, Transplantatabstoßung, Transplantat-Wirt-Reaktionen, Reperfusionsschäden, neurologischen Störungen und Eisenüberladung ausgewählt ist und das Verfahren das Kombinieren eines pharmazeutisch annehmbaren Exzipienten, der zur subkutanen Injektion geeignet ist, mit dem Mononatrium- oder Dinatriumsalz von N,N'-Bis(2-hydroxybenzyl)ethylendiamin-N,N'-diessigsäure umfasst.

## Revendications

1. Une composition pharmaceutique comprenant un excipient pharmaceutiquement acceptable approprié pour injection sous-cutanée en combinaison avec une quantité thérapeutiquement efficace du sel monosodique ou disodique de l'acide N,N'-bis(2-hydroxybenzyl)éthylènediamine-N,N'-diacétique.

2. La composition de la revendication 1, dans laquelle le composé est combiné à un excipient pharmaceutiquement acceptable en tant que solution injectable.

3. La composition de la revendication 1, dans laquelle le composé est combiné avec un excipient pharmaceutiquement acceptable en tant que suspension injectable.

4. La composition de la revendication 1, dans laquelle le composé est combiné avec un excipient pharmaceutiquement acceptable en tant que composition aqueuse.

5. La composition de la revendication 1, dans laquelle le composé est combiné avec un excipient pharmaceutiquement acceptable en tant que composition non-aqueuse.

6. Utilisation d'un excipient pharmaceutiquement acceptable approprié pour injection sous-cutané et de l'acide N,N'-bis(2-hydroxybenzyl)éthylènediamine-N,N'-diacétique ou d'un sel pharmaceutiquement acceptable de celui-ci dans la fabrication d'une composition pour traiter un mammifère présentant un état de maladie qui peut être traité par un agent de chélateur du fer et est choisi parmi la malaria, le cancer, les infections HIV, les infections inflammatoires touchant le tube disgestif, les rejets hôtes/greffes, les rejets greffes/hôtes, les lésions de reperfusion, les désordres neurologiques ainsi que la surcharge en fer, où une quantité thérapeutiquement efficace de la composition doit être administrée par injection sous-cutanée.

7. L'utilisation de la revendication 6, dans lequel l'état de maladie est une surcharge en fer.

8. L'utilisation de la revendication 7, dans lequel l'état de maladie est une hémochromatose.

9. L'utilisation de la revendication 7, dans lequel l'état de maladie est une hémochromatose secondaire.

10. L'utilisation de la revendication 7, dans lequel l'état de maladie est une hémosidérose focale.

11. Un objet de fabrication qui comprend un récipient contenant une composition pharmaceutique comprenant un excipient pharmaceutiquement acceptable approprié pour injection sous-cutanée en combinaison avec un sel monosodique ou disodique de l'acide N,N'-bis(2-hydroxybenzyl)éthylènediamine-N,N'-diacétique, où le récipient contient une quantité thérapeutiquement active du sel et est associé avec des instructions imprimées de marquage pour l'administration de la composition afin de traiter un état de maladie qui peut être traité par un chélateur du fer, ladite maladie étant choisie parmi la malaria, le cancer, les infections HIV, les infections inflammatoires touchant le tube disgestif, les rejets hôtes/greffes, les rejets greffes/hôtes, les lésions de reperfusion, les désordres neurologiques ainsi que la surcharge en fer.

12. L'objet de fabrication de la revendication 11, dans lequel l'état de maladie est un état de surcharge en fer qui est une hémochromatose primaire, une hémochromatose secondaire ou une hémosidérose focale.

13. L'objet de fabrication de la revendication 11, dans lequel le composé est combiné avec un excipient pharmaceutiquement acceptable en tant que solution injectable, suspension injectable, composition aqueuse ou composition non-aqueuse.

14. Un procédé de préparation d'une composition pharmaceutique, lequel procédé comprend la combinaison d'un excipient pharmaceutiquement acceptable approprié pour injection sous-cutanée avec le sel monosodique ou disodique de l'acide N,N-bis (2-hydroxybenzyl)éthylènediamine-N,N'-diacétique.

15. Le procédé de la revendication 14, dans lequel le sel est le sel disodique.

16. Le procédé de la revendication 14, dans lequel le sel est le sel monosodique.

17. Le procédé de la revendication 14, 15 ou 16, dans lequel le sel est combiné avec un excipient pharmaceutiquement acceptable en tant que solution injectable, suspension injectable, composition aqueuse ou composition non-aqueuse.

18. Le sel monosodique de l'acide N,N'-bis(2-hydroxybenzyl)éthylènediamine-N,N'-diacétique.

19. Le sel disodique de l'acide N,N'-bis(2-hydroxybenzyl)éthylènediamine-N,N'-diacétique.

20. Un procédé de préparation d'une composition utile pour traiter un mammifère présentant un état de maladie qui peut être traité par un chélateur du fer, ladite maladie étant choisie parmi la malaria, le cancer, les infections HIV, les infections inflammatoires touchant le tube disgestif, les rejets hôtes/greffes, les rejets greffes/hôtes, les lésions de reperfusion, les désordres neurologiques ainsi que la surcharge en fer, et lequel procédé comprend la combinaison d'un excipient pharmaceutiquement acceptable approprié pour injection sous-cutanée avec le sel monosodique ou disodique de l'acide N,N'-bis(2-hydroxybenzyl)éthylènediamine-N,N'-diacétique.
